# EUROPÄISCHE PATENTSCHRIFT

(11) **EP 1 102 974 B1**
(45) Veröffentlichungstag und Bekanntmachung des Hinweises auf die Patenterteilung: **07.05.2003**
(21) Anmeldenummer: 99941518.5
(22) Anmeldetag: 04.08.1999
(51) Int. Cl.: G01N 21/27, G01N 33/72

(54) **VORRICHTUNG UND VERFAHREN ZUM BESTIMMEN EINES HÄMOGLOBINWERTS VON BLUT**
DEVICE AND METHOD FOR DETERMINING THE HEMOGLOBIN VALUE IN BLOOD
APPAREIL ET PROCEDE PERMETTANT DE DETERMINER LE TAUX D'HEMOGLOBINE SANGUINE

(30) Priorität: 04.08.1998 DE 19835243
(43) Veröffentlichungstag der Anmeldung: 30.05.2001
(73) Patentinhaber: LMB Technologie GmbH, 85445 Schwaig (DE)
(72) Erfinder: JENTSCH, Klaus, D-85445 Schwaig (DE)
(74) Vertreter: Winter, Brandl & Partner
(86) Internationale Anmeldenummer: EP9905657
(87) Internationale Veröffentlichungsnummer: WO00008441

(56) Entgegenhaltungen:
- EP-A- 0 416 587
- WO-A-95/24651
- DE-A- 2 820 441
- DE-A- 3 439 181
- DE-A- 3 729 189

## Beschreibung

Die vorliegende Erfindung betrifft eine Vorrichtung und ein Verfahren zum Bestimmen eines Hämoglobinwerts von Blut.

Es ist bekannt, daß der Hämoglobinwert von Blut durch eine photometrische Bestimmung festgestellt werden kann. Bei einer derartigen photometrischen Bestimmung wird eine vorbestimmte Reagenzmenge mit einer vorbestimmten Blutmenge in einer Kammer, wie zum Beispiel einem Gefäß oder einer Küvette, zur Reaktion gebracht.

Dabei dauert eine Reaktion vom Zusammenbringen des Reagenz mit dem Blut bis zum Reaktionsendzeitpunkt ungefähr 60 Sekunden. Unterschiede der Dauer bis zum Erreichen des Reaktionsendzeitpunkts hängen dabei von dem jeweils vorliegenden Hämoglobinwert des Bluts ab. Je höher der Hämoglobinwert, das heißt, die Konzentration des Hämoglobins, im Blut ist, desto länger dauert es, bis der Reaktionsendzeitpunkt erreicht ist.

Nach Erreichen des Reaktionsendzeitpunkts wird der Hämoglobinwert mittels einer Lichtquelle und einer Erfassungseinrichtung bestimmt. Die Lichtquelle durchleuchtet dabei das sich aus der Reaktion ergebende Produkt und die Erfassungseinrichtung erfaßt das durch dieses Produkt hindurchgehende Licht und gibt ein dieses Licht und folglich den Hämoglobinwert anzeigendes Signal aus.

Die Messung des Hämoglobinwerts von Blut ist zum Beispiel bei der Blutspende wichtig. Es ist erforderlich, den Hämoglobinwert der möglichen Blutspender zu kennen, um zu erkennen, ob das Blut eines möglichen Blutspenders zur Spende geeignet ist oder nicht, und/oder um zu erkennen, welche Qualität das Blut des möglichen Blutspenders aufweist. Typischerweise weist ein gesunder Mensch einen Hämoglobinwert von ungefähr 13,4 bis 17,3 g/dl auf. Liegt der Hämoglobinwert des möglichen Spenders außerhalb des normalen Wertebereichs, wird dieser nicht zur Blutspende zugelassen. Die genauen Richtlinien bezüglich der Voraussetzungen zur Blutspende sind von der Deutschen Gesellschaft für Transfusionsmedizin und Immunhämatologie festgelegt worden, auf die an dieser Stelle verwiesen sei.

Die Messung des Hämoglobinwerts bei der Blutspende ist bisher an einer zentralen Stelle vor der Blutspende durchgeführt worden. Daraus ergibt sich das Problem von Wartezeiten an dieser zentralen Stelle, da jede einzelne Messung des Hämoglobinwerts ungefähr 60 Sekunden erfordert, bis das Endergebnis der Messung vorliegt und Klarheit darüber besteht, ob der mögliche Spender zur Blutspende geeignet ist oder nicht.

Dies führt dazu, daß die Möglichkeit besteht, daß es zu längeren Wartezeiten für die möglichen Blutspender kommt. Weiterhin führt dieses "Nadelöhr" dazu, daß mehrere Blutentnahmestellen für gewisse Zeiten frei bleiben, wodurch sich der gesamte Ablauf der Blutspendeaktion verzögert.

**Aus DE-A-34 39 181** ist eine Vorrichtung zur Auswertung von klinisch-chemischen Analysen mit zeitabhängigem Verlauf bekannt, die eine Kammer, in der eine vorbestimmte Blutmenge mit einer vorbestimmten Reagenzmenge zur Reaktion gebracht wird; eine Einrichtung zum photometrischen Bestimmen einer Konzentration eines Analyten durch Erfassen eines durch das Reaktionsprodukts gehenden Lichts vor dem Ende der Reaktion; eine Einrichtung zum Speichern eines diese Konzentration des Analyten darstellenden Werts vor dem Ende der Reaktion, wobei das photometrische Bestimmen und das Speichern eine vorbestimmte Anzahl von Zyklen und in vorbestimmten Abständern vor dem Ende der Reaktion wiederholt werden; und eine Einrichtung zum Voraussagen der Konzentration des Analyten nach dem Ende der Reaktion anhand der gespeicherten Werte vor dem Ende der Reaktion.

**Aus DE-A-34 39 181** ist ferner ein Verfahren zur Auswertung von klinisch-chemischen Analysen mit zeitabhängigem Verlauf bekannt, das die folgenden Schritte eines Bringens einer vorbestimmten Blutmenge mit einer vorbestimmten Reagenzmenge zu einer Reaktion; eines photometrischen Bestimmens einer Konzentration eines Analyten durch Erfassen eines durch das Reaktionsprodukt gehenden Lichts und eines Speicherns eines diese Konzentration des Analyten darstellenden Werts vor dem Ende der Reaktion; eines Wiederholens des Bestimmens und des Speicherns eine vorbestimmte Anzahl von Zyklen und in vorbestimmten Abständen vor dem Ende der Reaktion; und eines Voraussagens der Konzentration des Analyten nach dem Ende der Reaktion anhand der gespeicherten Werte vor dem Ende der Reaktion aufweist.

Es ist demgemäß die Aufgabe der vorliegenden Erfindung eine Vorrichtung und ein Verfahren zum Bestimmen des Hämoglobinwerts von Blut zu schaffen, mittels welchen der Hämoglobinwert von Blut schnell und dennoch zuverlässig bestimmt werden kann und die schnell in ein Gesamtsystem oder ähnliches integriert werden können. Diese Aufgabe der vorliegenden Erfindung wird hinsichtlich der Vorrichtung mit den im Anspruch 1 angegebenen Maßnahmen und hinsichtlich des Verfahrens mittels den im Anspruch 10 angegebenen Maßnahmen gelöst.

Erfindungsgemäß wird eine Vorrichtung geschaffen, die eine Kammer, in der eine vorbestimmte Blutmenge mit einer vorbestimmten Reagenzmenge zur Reaktion gebracht wird; eine Einrichtung zum photometrischen Bestimmen eines Hämoglobinwerts durch Erfassen eines durch das Reaktionsprodukt gehenden Lichts vor dem Ende der Reaktion; eine Einrichtung zum Speichern eines diesen Hämoglobinwert darstellenden Werts vor dem Ende der Reaktion, wobei das photometrische Bestimmen und das Speichern eine vorbestimmte Anzahl von Zyklen und in vorbestimmten Abständen vor dem Ende der Reaktion wiederholt werden; eine Einrichtung zum Voraussagen des Hämoglobinwerts nach dem Ende der Reaktion anhand der gespeicherten Werte vor dem Ende der Reaktion; und eine Einrichtung zum Übertragen eines vorausgesagten Reaktionsverlaufs und/oder des vorausgesagten Hämoglobinwerts nach dem Ende der Reaktion zu einer fernen Einheit aufweist.

Dadurch, daß die erfindungsgemäße Vorrichtung in der Lage ist, den vorliegenden Hämoglobinwert anhand der gespeicherten Werte bereits weit vor dem Reaktionsendzeitpunkt vorauszusagen, das heißt, hochzurechnen, kann der Hämoglobinwert sehr schnell erfaßt werden. Weiterhin ist es durch Untersuchungen festgestellt worden, daß ein sehr hoher Vertrauensbereich von ungefähr 95 Prozent oder mehr bezüglich des erzielten Ergebnisses besteht, so daß die erfindungsgemäße Vorrichtung sehr zuverlässig arbeitet.

Weiterhin besteht durch die Übertragung zu der fernen Einheit, die vorzugsweise drahtlos durchgeführt wird, auf einfache Weise die Möglichkeit, die erfindungsgemäße Vorrichtung in ein Gesamtsystem bei einer Blutspendeaktion oder ähnliches zu integrieren.

In einer weiteren bevorzugten Ausgestaltung der vorliegenden Erfindung ist die Vorrichtung zum Voraussagen eines Hämoglobinwerts von Blut in ein Blutentnahmegerät integriert vorgesehen, wodurch bei der Blutspende die Möglichkeit besteht, den Hämoglobinwert direkt an der Blutentnahmestelle zu messen, ohne daß zusätzlich eine getrennte Vorrichtung erforderlich ist.

Weiterhin wird gemäß der vorliegenden Erfindung ein Verfahren zum Bestimmen eines Hämoglobinwerts von Blut, das die folgenden Schritte eines Bringens einer vorbestimmten Blutmenge mit einer vorbestimmten Reagenzmenge zu einer Reaktion; eines photometrischen Bestimmens eines Hämoglobinwerts durch Erfassen eines durch das Reaktionsprodukt gehenden Lichts und eines Speicherns eines diesen Hämoglobinwert darstellenden Werts vor dem Ende der Reaktion; eines Wiederholens der Schritte des Bestimmens und des Speicherns eine vorbestimmte Anzahl von Zyklen und in vorbestimmten Abständen vor dem Ende der Reaktion; eines Voraussagens des Hämoglobinwerts nach dem Ende der Reaktion anhand der gespeicherten Werte vor dem Ende der Reaktion; und eines Übertragens eines vorausgesagten Reaktionsverlaufs und/oder des vorausgesagten Hämoglobinwerts nach dem Ende der Reaktion zu einer fernen Einheit aufweist.

Weitere vorteilhafte Ausgestaltungen der vorliegenden Erfindung sind Gegenstand der Unteransprüche.

Die vorliegende Erfindung wird nachstehend anhand eines Ausführungsbeispiels unter Bezugnahme auf die beiliegende Zeichnung näher erläutert.

Es zeigt:
- **Fig. 1**: ein Blockschaltbild einer Vorrichtung zum Bestimmen eines Hämoglobinwerts von Blut gemäß einem Ausführungsbeispiel der vorliegenden Erfindung;
- **Fig. 2**: eine in dem Ausführungsbeispiel verwendete Kurve; und
- **Fig. 3**: ein Flußdiagramm eines Verfahrensablaufs zum Bestimmen eines Hämoglobinwerts von Blut gemäß dem Ausführungsbeispiel der vorliegenden Erfindung.

Nachstehend erfolgt die Beschreibung eines Ausführungsbeispiels der vorliegenden Erfindung.

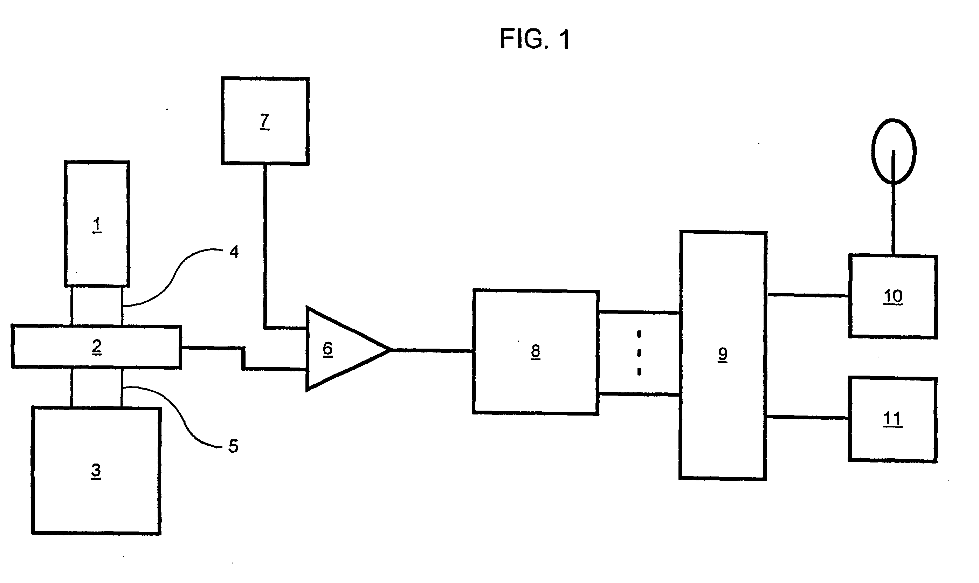

In Fig. 1 ist eine Vorrichtung zum Bestimmen eines Hämoglobinwerts von Blut gemäß dem Ausführungsbeispiel der vorliegenden Erfindung gezeigt.

In Fig. 1 bezeichnet das Bezugszeichen 1 eine Lichtquelle, bezeichnet das Bezugszeichen 2 eine Kammer, wie zum Beispiel eine Küvette, bezeichnet das Bezugszeichen 3 eine Erfassungseinrichtung, bezeichnet das Bezugszeichen 4 einen von der Lichtquelle abgegebenen Lichtstrahl, bezeichnet das Bezugszeichen 5 einen von der Erfassungseinrichtung aufgenommenen Lichtstrahl, bezeichnet das Bezugszeichen 6 einen als Vergleicher dienenden Verstärker, bezeichnet das Bezugszeichen 7 eine Referenzenergieversorgungsquelle, bezeichnet das Bezugszeichen 8 eine einen Speicher aufweisende Logikeinheit, bezeichnet das Bezugszeichen 9 eine Elektronik, bezeichnet das Bezugszeichen 10 eine Datenausgabeeinrichtung und bezeichnet das Bezugszeichen 11 eine Anzeige.

Nachstehend wird der in Fig. 1 gezeigte Aufbau und dessen Funktionsweise näher erläutert.

Wenn in die Kammer 2 eine vorbestimmte Blutmenge mit einer vorbestimmten Reagenzmenge zur Reaktion gebracht wird, bewirkt dies, daß sich die Farbgebung des sich ergebenden Reaktionsprodukts in Übereinstimmung mit dem in dem Blut enthaltenen Hämoglobinwert ändert. Wie es bereits zuvor beschrieben worden ist, ist die Reaktion nach ungefähr 60 Sekunden zu Ende, was bedeutet, daß nach diesen 60 Sekunden ein stabiler Wert vorliegt.

Die Lichtquelle 1 gibt einen Lichtstrahl 4 ab, der in die Kammer 2 einfällt. Ein durch die Kammer 2 hindurchgehender Lichtstrahl 5 wird dann von der Erfassungseinrichtung 3 erfaßt.

Die Erfassungseinrichtung 3 wandelt den aufgenommenen Lichtstrahl 5 in ein Signal, das ein die Intensität dieses Lichtstrahls 5 und demzufolge ein den Hämoglobinwert anzeigendes Signal darstellt.

Dieses Signal wird anhand des Verstärkers 6 mit einem von der Referenzenergieversorgungsquelle 7 gelieferten Signal verglichen. Diese Vorgehensweise ist im Stand der Technik bekannt und wird hier deshalb nicht näher erläutert.

Das derart erzielte Signal, das den Hämoglobinwert darstellt, wird der einen Speicher aufweisenden Logikeinheit 8 zugeführt und in dieser gespeichert.

Diese Logikeinheit 8 ist weiterhin mit einer Elektronik 9 verbunden, die dazu dient, daß noch weitere Zusatzgeräte zur Datenerfassung (wie zum Beispiel Probennummer, Chargennummer, usw.) und/oder Datenausgabe angeschlossen werden können.

In Fig. 1 ist an die Elektronik 9 eine Datenausgabeeinrichtung 10 angeschlossen, mittels der erzielte Meßergebnisse zu einer fernen Einheit, wie zum Beispiel einer Zentralstation, übertragen werden können. Die Übertragung kann dabei sowohl über Draht als auch auf eine andere Weise, wie zum Beispiel drahtlos, optisch, usw., erfolgen, wobei eine drahtlose Übertragung bevorzugt ist.

Weiterhin ist an die Elektronik 9 eine Anzeige 11 angeschlossen, mittels der erzielte Meßergebnisse, Alarmmeldungen oder ähnliches angezeigt werden können.

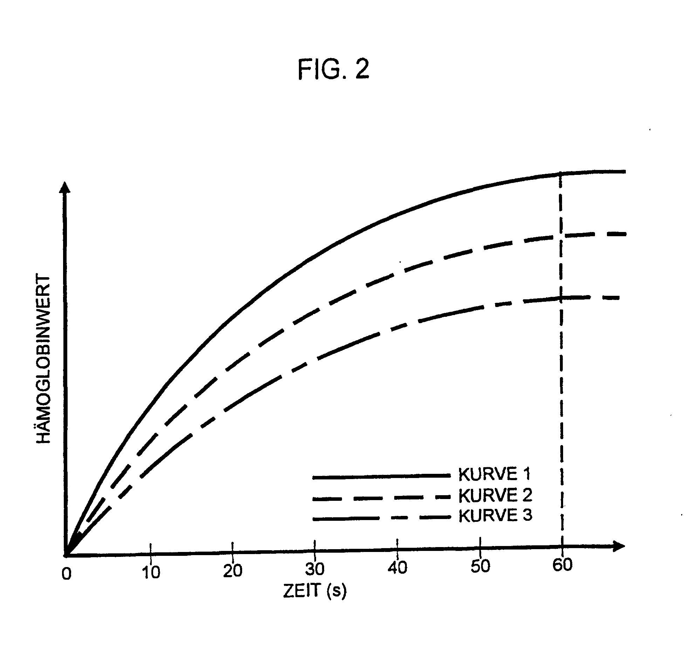

Es wird auf Fig. 2 verwiesen. Wenn das Blut mit einem Reagenz zur Reaktion gebracht wird, werden Verlaufskurven der Reaktion erzielt, wie sie schematisch in Fig. 2 gezeigt sind. Es ist zu sehen, daß die Kurven nach ungefähr 60 Sekunden einen stabilen Endwert aufweisen, was den Reaktionsendzeitpunkt des Bluts mit dem Reagenz anzeigt. Dieser stabile Endwert stellt den vorliegenden Hämoglobinwert des Bluts dar. Die Kurven weisen aufgrund der immer annähernd gleichen Konzentration des Reagenz dabei immer eine annähernd gleiche Form auf. Die in Fig. 2 gezeigten Kurvenformen weisen daher Unterschiede lediglich aufgrund des im Blut vorhandenen Hämoglobins bzw. Blutfarbstoffs auf. Gemäß der Darstellung in Fig. 2 zeigt daher die Kurve 1 den Reaktionsverlauf bei einem hohen Hämoglobinwert an, zeigt die Kurve 2 den Reaktionsverlauf bei einem mittleren Hämoglobinwert an und zeigt die Kurve 3 den Hämoglobinwert bei einem niedrigen Hämoglobinwert an.

Wie es in Fig. 2 zu sehen ist, weisen die Kurven bereits nach kurzer Zeit nach dem Beginn einer Reaktion deutliche Unterschiede auf, die durch den im Blut vorhandenen Hämoglobinwert bestimmt werden. Erfindungsgemäß ist es festgestellt worden, daß aufgrund von mehreren Hämoglobinwerten, die nach kurzer Zeit nach Beginn der Reaktion erfaßt werden, der Hämoglobinwert nach dem Ende der Reaktion vorausgesagt werden kann. Dies wird nachstehend detaillierter beschrieben.

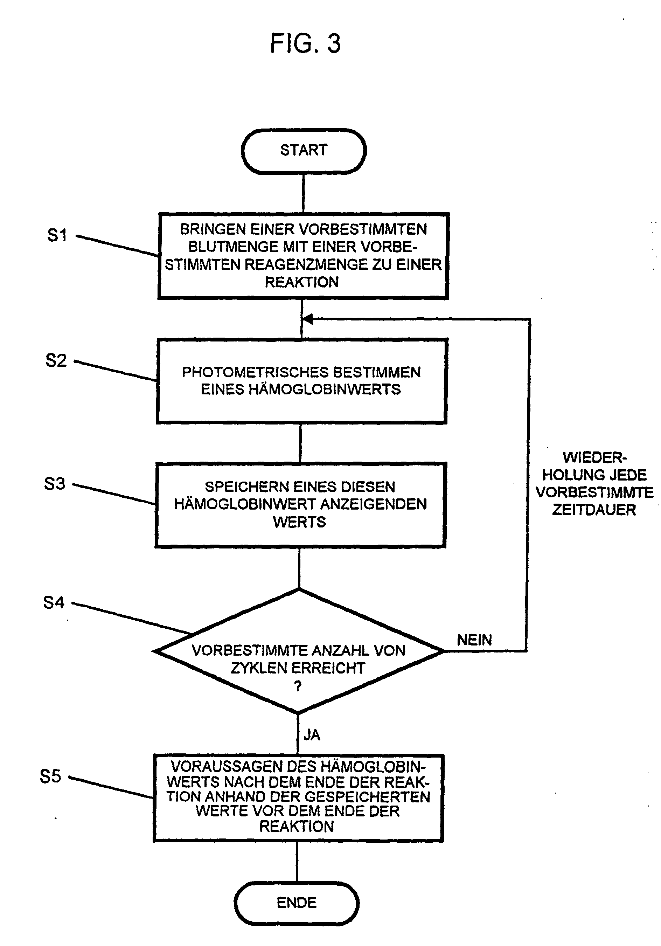

Wie es in Fig. 3 gezeigt ist, wird von der in Fig. 1 gezeigten Vorrichtung der folgende grundlegende Verfahrensablauf durchgeführt.

In einem Schritt S1 wird die vorbestimmte Blutmenge mit der vorbestimmten Reagenzmenge zur Reaktion gebracht. Dies wird innerhalb der Kammer 2 durchgeführt. In einem dem Schritt 1 nachfolgenden Schritt S2 wird der Hämoglobinwert, das heißt, der aktuell vorliegende Wert, der der Farbgebung des Reaktionsprodukts entspricht, mittels der Lichtquelle 1 und der Erfassungseinrichtung 3 photometrisch bestimmt. Im Anschluß daran wird in einem Schritt S3 ein den derart erfaßten Hämoglobinwert anzeigender Wert in der Logikeinheit 8 abgespeichert.

In einem Schritt S4 wird es nachfolgend bestimmt, ob die zuvor beschriebenen Schritte S2 und S3 eine vorbestimmte Anzahl von Zyklen durchgeführt worden sind. Die Anzahl der vorbestimmten Zyklen beträgt vorzugsweise zwischen 15 und 20, kann aber je nach vorliegendem Anwendungsfall und/oder erforderlicher Zuverlässigkeit zweckmäßig festgelegt werden.

Wenn im Schritt S4 eine negative Antwort (NEIN) erzielt wird, werden die Schritte S2 und S3 solange wiederholt, bis im Schritt S4 eine positive Antwort (JA) erzielt wird. Die Schritte S2 und S3 werden dabei in einer vorbestimmten Zeitdauer wiederholt. In diesem Ausführungsbeispiel der vorliegenden Erfindung beträgt die Zeitdauer, in der die Schritte S2 und S3 wiederholt werden, eine Sekunde. Die Zeitdauer kann jedoch je nach vorliegendem Anwendungsfall und/oder erforderlicher Zuverlässigkeit zweckmäßig festgelegt werden.

Es ist anzumerken, daß in diesem Ausführungsbeispiel der vorliegenden Erfindung die vorbestimmte Anzahl von Zyklen und die vorbestimmte Zeitdauer der Wiederholung der Schritte S2 und S3 derart eingestellt werden, daß im Schritt S4 eine positive Antwort (S4) erzielt wird, deutlich bevor der Reaktionsendzeitpunkt erreicht ist.

Wird nach Erreichen der vorbestimmten Anzahl von Zyklen im Schritt S4 eine positive Antwort (JA) erzielt, wird in einem Schritt S5 der Hämoglobinwert nach dem Ende der Reaktion, das heißt, der tatsächliche im Blut vorhandene Hämoglobinwert, anhand der im Schritt S3 gespeicherten Werte vorausgesagt bzw. hochgerechnet.

Diese Voraussage kann zum Beispiel anhand einer Standardkurve erfolgen, die in der Logikeinheit 8 gespeichert ist, da der Reaktionsverlauf, wie es bereits zuvor beschrieben worden ist, lediglich von dem im Blut vorhandenen Hämoglobin abhängt, wenn die Konzentration des Reagenz, mit dem das Blut zur Reaktion gebracht wird, immer annähernd gleich ist.

Ebenso besteht die Möglichkeit, Referenzwerte in der Logikeinheit 8 zu speichern, die Reaktionsverläufe für jeweilige Hämoglobinwerte angeben, und diese Referenzwerte mit den jeweils von der Erfassungseinrichtung 3 erfaßten Werten zu vergleichen.

Aufgrund der vorstehend genannten Verfahrensweise besteht demgemäß die Möglichkeit, den Hämoglobinwert von zu untersuchendem Blut in einer Zeitdauer (15 bis 20 Sekunden) festzustellen, die deutlich kürzer als die Zeitdauer ist, die bis zum Reaktionsendzeitpunkt (ungefähr 60 Sekunden) verstreicht.

Untersuchungen haben ergeben, daß es, wenn der Hämoglobinwert unter Zuhilfenahme von ungefähr 15 bis 20 Werten, die in einem Abstand von jeweils einer Sekunde erfaßt werden, möglich ist, den Hämoglobinwert mit einer Zuverlässigkeit von 95 Prozent oder mehr richtig zu erfassen. Demgemäß kann der Hämoglobinwert nach dem Ende der Reaktion bereits nach 15 bis 20 Sekunden (anstatt nach 60 Sekunden, das heißt, dem Reaktionsendzeitpunkt) mit einer hohen Zuverlässigkeit ermittelt werden.

Es wird erneut auf Fig. 1 verwiesen. Der wie zuvor beschriebene vorausgesagte Hämoglobinwert und/oder die gesamte Verlaufskurve, die von der Logikeinheit vorausgesagt werden, können in der Logikeinheit 8 gespeichert werden. Weiterhin besteht die Möglichkeit, den Hämoglobinwert und/oder die gesamte Verlaufskurve aus der Logikeinheit 8 zu der Elektronik 9 auszugeben. Die Elektronik 9 kann dann den Hämoglobinwert und/oder die gesamte Verlaufskurve zu einer Anzeige 11 ausgeben, an der diese angezeigt werden.

In der Logikeinheit 8 können ebenso Kurven von obersten und untersten Grenzen des Hämoglobinwerts gespeichert sein. Wird es im Verlauf des vorstehend genannten Verfahrensablaufs festgestellt, daß die erfaßten Werte die oberste Kurve überschreiten oder die unterste Kurve unterschreiten, was bedeutet, daß das Blut eines möglichen Blutspenders nicht zur Blutspende geeignet ist, kann ein Alarm ausgegeben werden. Dieser Alarm kann zum Beispiel akustisch abgegeben werden und/oder kann zum Beispiel an der Anzeige 11 angezeigt werden.

Ebenso besteht die Möglichkeit, den Hämoglobinwert und/oder die gesamte Verlaufskurve aus der Logikeinheit 8 zu einer Datenausgabeeinrichtung 10 auszugeben, mittels der dann den Hämoglobinwert und/oder die Verlaufskurve anzeigende Daten zu einer fernen Einheit übertragen werden können. Diese ferne Einheit kann zum Beispiel eine bei einer Blutspendeaktion erforderliche Zentralstation sein, in der alle erforderlichen Daten gespeichert werden. Derartige Daten beinhalten unter anderem zum Beispiel alle spenderspezifischen Daten. Dabei ist es bevorzugt, daß die Übertragung drahtlos, wie zum Beispiel per Funk, erfolgt. Es kann jedoch auch eine drahtgebundene Übertragung, eine optische Übertragung, usw. durchgeführt werden.

Wie es aus der vorhergehenden Beschreibung ersichtlich ist, besteht erfindungsgemäß die Möglichkeit, einen Hämoglobinwert von Blut nach einer Zeit, die deutlich vor dem Erreichen des Reaktionsendzeitpunkts liegt, mit einer hohen Zuverlässigkeit zu ermitteln.

Demgemäß können bei einer Blutspendeaktion lange Wartezeiten an einer zentralen Stelle, an der die Spendefähigkeit, das heißt, unter anderem der Hämoglobinwert, des möglichen Spenders festgestellt wird, deutlich zu verkürzen. Ebenso besteht die Möglichkeit, die zuvor beschriebene Vorrichtung nicht zentral sondern dezentral an jeder Blutentnahmestelle vorzusehen, so daß bereits während des Erfassens des Hämoglobinwerts mit den Vorbereitungen zur Blutspende begonnen werden kann. In der Praxis ist dadurch insgesamt ein flüssigerer Verfahrensablauf der Blutspendeaktion möglich.

Sollte es festgestellt werden, daß der mögliche Spender einen zur Blutspende ungeeigneten Hämoglobinwert aufweist, kann zum Beispiel die Messung bis zum Erreichen des Reaktionsendzeitpunkts fortgeführt werden und kann dann endgültig über die Eignung des möglichen Spenders entschieden werden. Somit wird eine in wenigen Fällen vorliegende fehlerhafte Voraussage des Hämoglobinwerts kompensiert. Da die meisten Spender einen zur Spende geeigneten Hämoglobinwert aufweisen und dieser in aller Regel auch richtig vorausgesagt wird, ist dadurch eine sehr zügige Abwicklung der Blutspendeaktion möglich.

Ein erheblicher Vorteil wird erzielt, wenn die zuvor beschriebene Vorrichtung nicht als getrennte Vorrichtung sondern in ein für die Blutspende verwendetes Blutentnahmegerät integriert ausbildet wird. Wenn dies der Fall ist, kann mit dem Blutentnahmegerät die Erfassung des Hämoglobinwerts durchgeführt werden, während gleichzeitig mit den Vorbereitungen für die Blutspende begonnen wird, ohne daß es erforderlich ist, eine weitere Vorrichtung zusätzlich zu dem Blutentnahmegerät zu verwenden. Ein derartiges Blutentnahmegerät, in das die erfindungsgemäße Vorrichtung integriert werden kann, wird zum Beispiel von der Anmelderin der vorliegenden Erfindung unter der Bezeichnung BAGMATIC vertrieben. In dem Fall des Integrierens der erfindungsgemäßen Vorrichtung in das Blutentnahmegerät besteht weiterhin die Möglichkeit, bestimmte Einrichtungen, wie zum Beispiel die Datenausgabeeeinrichtung 10, die Anzeige 11, usw., nicht getrennt für die Vorrichtung vorzusehen, sondern bereits vorhandene Einrichtungen des Blutentnahmegeräts mit zu verwenden.

Vorstehend sind eine Vorrichtung und ein Verfahren offenbart worden, mittels denen der Hämoglobinwert von Blut innerhalb einer kurzen Zeitdauer vor dem Reaktionsendzeitpunkt eines Reagenz mit dem Blut anhand einer Voraussage bzw. Hochrechnung zur Verfügung gestellt werden kann. Es ist darauf zu verweisen, daß, obwohl es zuvor beschrieben worden ist, das Verfahren und die Vorrichtung bei der Blutspende anzuwenden, es jedoch ebenso möglich ist, das Verfahren und die Vorrichtung in anderen Anwendungsfällen einzusetzen, bei denen es erforderlich ist, den Hämoglobinwert von Blut zu ermitteln.

## Patentansprüche

1. Vorrichtung zum Bestimmen eines Hämoglobinwerts von Blut, die aufweist:
**[a]** eine Kammer (2), in der eine vorbestimmte Blutmenge mit einer vorbestimmten Reagenzmenge zur Reaktion gebracht wird;
**[b]** eine Einrichtung (1, 3) zum photometrischen Bestimmen eines Hämoglobinwerts durch Erfassen eines durch das Reaktionsprodukt gehenden Lichts vor dem Ende der Reaktion;
**[c]** eine Einrichtung (8) zum Speichern eines diesen Hämoglobinwert darstellenden Werts vor dem Ende der Reaktion, wobei
das photometrische Bestimmen und das Speichern eine vorbestimmte Anzahl von Zyklen und in vorbestimmten Abständen vor dem Ende der Reaktion wiederholt werden;
**[d]** eine Einrichtung (8) zum Voraussagen des Hämoglobinwerts nach dem Ende der Reaktion anhand der gespeicherten Werte vor dem Ende der Reaktion; und
**[e]** eine Einrichtung (10) zum Übertragen eines vorausgesagten Reaktionsverlaufs und/oder des vorausgesagten Hämoglobinwerts nach dem Ende der Reaktion zu einer fernen Einheit.

2. Vorrichtung nach Anspruch **1, *dadurch gekennzeichnet,* *daß*** die Übertragung drahtlos durchgeführt wird.

3. Vorrichtung nach Anspruch 1 oder 2, ***gekennzeichnet durch*** eine Einrichtung (8) zum Speichern einer Standardkurve, die einen Reaktionsverlauf angibt, wobei das Voraussagen des Hämoglobinwerts anhand der gespeicherten Standardkurve durchgeführt wird.

4. Vorrichtung nach Anspruch 1 oder 2, ***gekennzeichnet durch*** eine Einrichtung (8) zum Speichern von Referenzwerten, die einen Reaktionsverlauf für unterschiedliche Hämoglobinwerte angeben, wobei das Voraussagen des Hämoglobinwerts anhand eines Vergleichs mit den gespeicherten Referenzwerten durchgeführt wird.

5. Vorrichtung nach einem der Ansprüche 1 bis 4, ***gekennzeichnet durch*** eine Einrichtung (8) zum Speichern von Kurven von obersten und untersten Grenzen des Hämoglobinwerts und eine Einrichtung (11), die bei Überschreiten der obersten Grenze und/oder Unterschreiten der untersten Grenze eine darüber unterrichtende Meldung abgibt.

6. Vorrichtung nach einem der Ansprüche 1 bis 5, ***dadurch gekennzeichnet, daß*** die vorbestimmten Abstände jeweils eine Sekunde betragen.

7. Vorrichtung nach einem der Ansprüche 1 bis 6, ***dadurch gekennzeichnet, daß*** sich die Anzahl der vorbestimmten Zyklen in einem Bereich von 15 bis 20 befindet.

8. Vorrichtung nach einem der Ansprüche 1 bis 7, ***gekennzeichnet durch*** eine Anzeige (11), die einen vorausgesagten Reaktionsverlauf und/oder den vorausgesagten Hämoglobinwert nach dem Ende der Reaktion anzeigt.

9. Blutentnahmegerät, ***dadurch gekennzeichnet, daß*** es eine Vorrichtung zum Bestimmen eines Hämoglobinwerts nach einem der Ansprüche 1 bis 8 aufweist.

10. Verfahren zum Bestimmen eines Hämoglobinwerts von Blut, das die folgenden Schritte aufweist:
**[1]** Bringen einer vorbestimmten Blutmenge mit einer vorbestimmten Reagenzmenge zu einer Reaktion;
**[2]** photometrisches Bestimmen eines Hämoglobinwerts durch Erfassen eines durch das Reaktionsprodukt gehenden Lichts und Speichern eines diesen Hämoglobinwert darstellenden Werts vor dem Ende der Reaktion;
**[3]** Wiederholen des Schritts **[2]** eine vorbestimmte Anzahl von Zyklen und in vorbestimmten Abständen vor dem Ende der Reaktion;
**[4]** Voraussagen des Hämoglobinwerts nach dem Ende der Reaktion anhand der gespeicherten Werte vor dem Ende der Reaktion; und
**[5]** Übertragen eines vorausgesagten Reaktionsverlaufs und/oder des vorausgesagten Hämoglobinwerts nach dem Ende der Reaktion zu einer fernen Einheit.

11. Verfahren nach Anspruch 10, ***dadurch gekennzeichnet, daß*** die Übertragung drahtlos durchgeführt wird.

12. Verfahren nach Anspruch 10 oder 11, ***dadurch gekennzeichnet, daß*** das Voraussagen des Hämoglobinwerts anhand einer gespeicherten Standardkurve durchgeführt wird, die einen Reaktionsverlauf angibt.

13. Verfahren nach Anspruch 10 oder 11, ***dadurch gekennzeichnet, daß*** das Voraussagen des Hämoglobinwerts anhand eines Vergleichs mit gespeicherten Referenzwerten durchgeführt wird, die einen Reaktionsverlauf für unterschiedliche Hämoglobinwerte angeben.

14. Verfahren nach einem der Ansprüche 10 bis 13, ***dadurch gekennzeichnet, daß*** Kurven von obersten und untersten Grenzen des Hämoglobinwerts gespeichert sind und bei Überschreiten der obersten Grenze und/oder Unterschreiten der untersten Grenze eine darüber unterrichtende Meldung abgegeben wird.

15. Verfahren nach einem der Ansprüche 10 bis 14, ***dadurch gekennzeichnet, daß*** die vorbestimmten Abstände jeweils eine Sekunde betragen.

16. Verfahren nach einem der Ansprüche 10 bis 15, ***dadurch gekennzeichnet, daß*** sich die Anzahl der vorbestimmten Zyklen in einem Bereich von 15 bis 20 befindet.

17. Verfahren nach einem der Ansprüche 10 bis 16, ***dadurch gekennzeichnet, daß*** ein vorausgesagter Reaktionsverlauf und/oder der vorausgesagte Hämoglobinwert nach dem Ende der Reaktion angezeigt wird.

## Claims

1. Device for determining the haemoglobin value of blood, comprising:
**[a]** a chamber (2) in which a predetermined blood quantity is reacted with a predetermined reagent quantity;
**[b]** means (1, 3) for photometrically determining the haemoglobin value by detecting light passing through the reaction product prior to the end of the reaction;
**[c]** means (8) for storing a value representing this haemoglobin value prior to the end of the reaction,
said photometric determination and storing being repeated a predetermined number of cycles and at predetermined intervals prior to the end of the reaction;
**[d]** means (8) for predicting, prior to the end of the reaction, the haemoglobin value after the end of the reaction from the stored values; and
**[e]** means (10) for transmitting the predicted course of the reaction and/or the predicted haemoglobin value after the end of the reaction to a remote unit.

2. Device according to claim 1, ***characterized in that*** the transmission is performed in a wireless manner.

3. Device according to one of claims 1 or 2, ***characterized by*** means (8) for storing a standard graph indicating the course of the reaction, the prediction of the haemoglobin value being carried out using the stored standard graph.

4. Device according to one of claims 1 or 2, ***characterized by*** means (8) for storing reference values indicating the course of the reaction for various haemoglobin values, the prediction of the haemoglobin value being carried out by making a comparison with the stored reference values.

5. Device according to one of claims 1 through 4, ***characterized by*** means (8) for storing graphs of upper and lower limits of the haemoglobin value, and means (11) outputting, in case the upper limit is exceeded and/or the lower limit is fallen below, an alarm instructing thereof.

6. Device according to one of claims 1 through 5, ***characterized in that*** the predetermined intervals each are one second.

7. Device according to one of claims 1 through 6, ***characterized in that*** the number of predetermined cycles is in the range of 15 to 20.

8. Device according to one of claims 1 through 7, ***characterized by*** a display (11) displaying a predicted course.of the reaction and/or the predicted haemoglobin value after the end of the reaction.

9. Device for taking samples of blood, ***characterized in* that** it comprises a device for determining the haemoglobin value in accordance with one of claims 1 through 8.

10. Method for determining the haemoglobin value of blood, comprising the following steps:
**[1]** reacting a predetermined blood quantity with a predetermined reagent quantity;
**[2]** photometrically determining the haemoglobin value by detecting light passing through the reaction product, and storing a value representing this haemoglobin value prior to the end of the reaction;
**[3]** repeating step **[2]** a predetermined number of cycles and at predetermined intervals prior to the end of the reaction;
**[4]** predicting, prior to the end of the reaction, the haemoglobin value after the end of the reaction using the stored values; and
**[5]** transmitting the predicted course of the reaction and/or the predicted haemoglobin value after the end of the reaction to a remote unit.

11. Method according to claim 10, ***characterized in that*** the transmission is carried out in a wireless manner.

12. Method according to one of claims 10 or 11, ***characterized in that*** the prediction of the haemoglobin value is effected using a stored standard graph indicating the course of the reaction.

13. Method according to one of claims 10 or 11, ***characterized in that*** the prediction of the haemoglobin value is effected by way of a comparison with stored reference values indicating the course of the reaction for various haemoglobin values.

14. Method according to one of claims 10 through 13, ***characterized in that*** graphs of upper and lower limits of the haemoglobin value are stored and that, in case the upper limit is exceeded and/or the lower limit is fallen below, an alarm instructing thereof is output.

15. Method according to one of claims 10 through 14, ***characterized in that*** said predetermined intervals each are one second.

16. Method according to one of claims 10 through 15, ***characterized in that*** the number of said predetermined cycles is in the range of 15 to 20.

17. Method according to one of claims 10 through 16, ***characterized in that*** a predicted course of the reaction and/or the predicted haemoglobin value is displayed after the end of the reaction.

## Revendications

1. Dispositif pour la détermination de la teneur en hémoglobine du sang comportant :
a) une chambre (2) , dans laquelle une quantité prédéterminée de sang est amenée avec une quantité prédéterminée de réactif pour réaliser la réaction ;
b) un dispositif (1, 3) pour la détermination photométrique de la teneur en hémoglobine grâce à la détection de la lumière émise par le produit de la réaction avant la fin de la réaction ;
c) un dispositif (8) pour enregistrer la valeur représentant cette teneur en hémoglobine avant là fin de la réaction ;
la détermination photométrique et l'enregistrement étant répétés pendant un nombre prédéterminé de cycles et à des intervalles prédéterminés avant la fin de la réaction ;
d) un dispositif (8) pour la prédiction de la teneur en hémoglobine après la fin de la réaction à l'aide des valeurs enregistrées avant la fin de la réaction ; et
e) un dispositif (10) pour la transmission d'un déroulement prévu de la réaction et/ou de la teneur prévue en hémoglobine après la fin de la réaction vers une unité distante.

2. Dispositif selon la revendication 1, **caractérisé en ce que** la transmission est réalisée sans fil:

3. Dispositif selon la revendication 1 ou 2, **caractérisé par** un dispositif (8) pour l'enregistrement d'une courbe standard qui donne le déroulement d'une réaction, la prédiction de la teneur en hémoglobine étant réalisée à l'aide de la courbe standard enregistrée.

4. Dispositif selon la revendication 1 ou 2, **caractérisé par** un dispositif (8) pour l'enregistrement de valeurs de référence qui indiquent un déroulement de la réaction pour différentes teneurs en hémoglobine, la prédiction de la teneur en hémoglobine étant réalisée à l'aide d'une comparaison avec les valeurs de référence enregistrées.

5. Dispositif selon l'une des revendications 1 à 4, **caractérisé par** un dispositif (8) pour l'enregistrement de courbes des limites supérieures et inférieures de la teneur en hémoglobine et par un dispositif (11) qui génère un message lors du dépassement de la limite supérieure et/ou du passage en dessous de la valeur inférieure.

6. Dispositif selon l'une des revendications 1 à 5, **caractérisé en ce que** les intervalles prédéterminés sont d'une seconde.

7. Dispositif selon l'une des revendications 1 à 6, **caractérisé en ce que** le nombre prédéterminé de cycles se situe entre 15 et 20.

8. Dispositif selon l'une des revendications 1 à 7, **caractérisé par** un affichage (11) qui affiche un déroulement de la réaction et/ou la teneur prévue en hémoglobine après la fin de la réaction.

9. Appareil de prélèvement de sang, **caractérisé en ce qu'**il comporte un dispositif pour déterminer une teneur en hémoglobine selon l'une des revendications 1 à 8.

10. Procédé de détermination de la teneur en hémoglobine du sang, comportant les étapes suivantes :
1) apport d'une quantité prédéterminée de sang avec une quantité prédéterminée de réactif pour une réaction ;
2) détermination photométrique de la teneur en hémoglobine par la détection de la lumière émise par le produit de la réaction et enregistrement de la valeur représentant cette teneur en hémoglobine avant la fin de la réaction ;
3) répétition de l'étape 2 pendant un nombre prédéterminé de cycles et à des intervalles prédéterminés avant la fin de la réaction ;
4) prédiction de la teneur en hémoglobine après la fin de la réaction à l'aide des valeurs enregistrées avant la fin de la réaction ; et
5) transmission du déroulement prévu de la. réaction et/ou de la teneur prévue en hémoglobine après la fin de la réaction vers une unité distante.

11. Procédé selon la revendication 10, **caractérisé en ce que** la transmission est réalisée sans fil.

12. Procédé selon la revendication 10 ou 11, **caractérisé en ce que** la prédiction de la teneur en hémoglobine est réalisée à l'aide d'une courbe standard enregistrée qui indique le déroulement de la réaction.

13. Procédé selon la revendication 10 ou 11, **caractérisé en ce que** la prédiction de la teneur en hémoglobine est réalisée à l'aide d'une comparaison avec des valeurs de référence enregistrées qui indiquent un déroulement de la réaction pour différentes teneurs en hémoglobine.

14. Procédé selon l'une des revendications 10 à 13, **caractérisé en ce que** les courbes des limites supérieures et inférieures de la teneur en hémoglobine sont enregistrées et **en ce qu'**un message, indiquant le dépassement de la limite supérieure et/ou le passage en dessous de la limite inférieure, est généré.

15. Procédé selon l'une des revendications 10 à 14, **caractérisé en ce que** les intervalles prédéterminés sont d'une seconde.

16. Procédé selon l'une des revendications 10 à 15, **caractérisé en ce que** le nombre prédéterminé de cycles se situe entre 15 et 20.

17. Procédé selon l'une des revendications 10 à 16, **caractérisé en ce qu'**un déroulement prévu de la réaction et/ou la teneur prédite en hémoglobine est affichée après la fin de la réaction.
